# EUROPEAN PATENT SPECIFICATION

(11) **EP 3 186 635 B1**
(45) Date of publication and mention of the grant of the patent: **07.08.2019**
(21) Application number: 15835338.3
(22) Date of filing: 31.08.2015
(51) Int. Cl.: G01N 33/558, G01N 33/53, G01N 33/52

(54) **A TEST STRIP FOR PAPER-BASED ASSAY**
TESTSTREIFEN FÜR TEST AUF PAPIERBASIS
BANDELETTE RÉACTIVE POUR ANALYSE À BASE DE PAPIER

(30) Priority: 30.08.2014 SG 10201405360P
(43) Date of publication of application: 05.07.2017
(73) Proprietor: Agency for Science, Technology And Research, Singapore 138632 (SG)
(72) Inventor: YING, Jackie Y., Singapore 138669 (SG); BAI, Jianhao, Singapore 138669 (SG); LEE, Yong Yeow, . (SG)
(74) Representative: Fabry, Bernd
(86) International application number: PCT/SG2015/050291
(87) International publication number: WO 2016/032404

(56) References cited:
- EP-A1- 1 566 640
- WO-A1-2014/085926
- WO-A2-2011/069029
- WO-A2-2013/040435
- JP-A- 2009 258 136
- US-A- 4 956 302
- US-A1- 2003 045 001
- US-A1- 2008 090 305

## Description

### FIELD OF THE INVENTION

The invention relates to a test strip for paper-based assays. The invention further relates to a method of detecting an analyte in a sample using said test strip.

### BACKGROUND OF THE INVENTION

Paper-based assays such as the dipstick and lateral flow tests are available as low-cost screening devices for many physiological-relevant compounds in bodily fluids. Paper-based assays utilize test strips that rely on the use of enzymes or bio/chemistry principles to enable detections of analytes or nucleotides or antibodies conjugated to detector molecules for analyte detection.

An important limitation of test strips used in lateral flow tests is the sensitivity of the test strip due to the poor limit-of-detection of the detector molecules used to visualize the results. Poor limit-of-detection translates to a larger sample requirement. While this limitation can be overcome via using the enzyme-substrate or horseradish peroxidase (HRP) with tetramethylbenzidine (TMB) amplification approach, a minimum waiting time is required prior to activation of the substrate addition step. As such, this introduces the possibility of human error. If the user activates the addition step too early, false negatives can arise. Another limitation of the enzyme-substrate amplification approach is the rate at which the enzymatic substrate contacts the test zone, which directly affects the rate of transport of the substrate to the enzymes and therefore assay sensitivity.

There is therefore a need to provide a test strip that overcomes, or at least ameliorates, one or more of the disadvantages described above.

### SUMMARY OF THE INVENTION

In one aspect, there is provided a test strip for a paper-based assay comprising, a substrate addition zone for receiving a substrate and a test zone, said test zone comprising a capture agent, wherein the substrate addition zone is located upstream of said test zone.

In one aspect, there is provided a paper-based enzyme assay comprising a test strip as described herein.

In one aspect, there is provided a method of detecting the presence of an analyte in a sample, said method comprising the steps of: a) contacting said sample onto said test zone of a test strip as described herein; b) dispensing a substrate onto the substrate addition zone; and c) detecting an emitted signal from said test zone to confirm the presence or absence of said analyte in said sample.

In one aspect, there is provided a method of detecting the presence of an analyte in a sample, said method comprising the steps of: a) contacting said sample onto said detector zone of a test strip as described herein; b) dispensing a substrate onto the substrate addition zone; and c) detecting an emitted signal from said test zone to confirm the presence or absence of said analyte in said sample.

### DEFINITIONS

The term "antigen binding protein" as used herein refers to antibodies, antibody fragments and other protein constructs, such as domains, which are capable of binding to an antigen.

The term "antibody" is used herein in the broadest sense to refer to molecules with an immunoglobulin-like domain and includes monoclonal, recombinant, polyclonal, chimeric, humanised, bispecific and heteroconjugate antibodies; a single variable domain, a domain antibody, antigen binding fragments, immunologically effective fragments, single chain Fv, diabodies, Tandabs™, etc (for a summary of alternative "antibody" formats see Holliger and Hudson, Nature Biotechnology, 2005, Vol 23, No. 9, 1126-1136).

The phrase "single variable domain" refers to an antigen binding protein variable domain (for example, V_{H}, V_{HH}, V_{L}) that specifically binds an antigen or epitope independently of a different variable region or domain.

A "domain antibody" or "dAb" may be considered the same as a "single variable domain" which is capable of binding to an antigen. A single variable domain may be a human antibody variable domain, but also includes single antibody variable domains from other species such as rodent, nurse shark and *Camelid* V_{HH} dAbs. Camelid V_{HH} are immunoglobulin single variable domain polypeptides that are derived from species including camel, llama, alpaca, dromedary, and guanaco, which produce heavy chain antibodies naturally devoid of light chains. Such V_{HH} domains may be humanised according to standard techniques available in the art, and such domains are considered to be "domain antibodies". As used herein V_{H} includes camelid V_{HH} domains.

As used herein the term "domain" refers to a folded protein structure which has tertiary structure independent of the rest of the protein. Generally, domains are responsible for discrete functional properties of proteins, and in many cases may be added, removed or transferred to other proteins without loss of function of the remainder of the protein and/or of the domain. A "single variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain. A domain can bind an antigen or epitope independently of a different variable region or domain.

An antigen binding fragment may be provided by means of arrangement of one or more CDRs on non-antibody protein scaffolds such as a domain. The domain may be a domain antibody or may be a domain which is a derivative of a scaffold selected from the group consisting of CTLA-4, lipocalin, SpA, an Affibody, an avimer, GroEl, transferrin, GroES and fibronectin/adnectin, which has been subjected to protein engineering in order to obtain binding to an antigen, other than the natural ligand.

An antigen binding fragment or an immunologically effective fragment may comprise partial heavy or light chain variable sequences. Fragments are at least 5, 6, 8 or 10 amino acids in length. Alternatively the fragments are at least 15, at least 20, at least 50, at least 75, or at least 100 amino acids in length.

The term "specifically binds" as used throughout the present specification in relation to antigen binding proteins means that the antigen binding protein binds to a specific antigen of interest with no or insignificant binding to other proteins. However, the term does not exclude the fact that the antigen binding proteins may also be cross-reactive with closely related molecules. The antigen binding proteins described herein may bind to an antigen with at least 2, 5, 10, 50, 100, or 1000 fold greater affinity than they bind to closely related molecules.

As used herein, the term "analyte" refers to a compound of interest. It will be generally understood that an analyte includes but is not limited to an antigen, antibody, hormone, drug, therapeutic, cell protein, nucleic acid, cardiac marker, tumor or cancer marker, autoimmune disease marker, or any macromolecule. For example, an antigen analyte can be an antigen associated with an infectious agent such as a virus, a bacterium, a fungus, or a prion. An example of a hormone analyte includes but is not limited to hCG, thyroxine, TSH, glucagons, insulin, relaxin, prolactin, luteinizing hormone, melanotropin, somatotropin, follicle-stimulating hormone, gastrin, bradykinin, vasopressin, and other releasing factors; other hormones of physiological or pathological interest can be the analyte. An example of an analyte that is a cancer or tumor marker includes but is not limited to prostate specific antigen (PSA), carcinoembryonic antigen (CEA), and α-fetoprotein. However, other cancer or tumor markers can be the analyte. An example of a drug analyte includes but is not limited to an opiate. Examples of an antibody analyte include but are not limited to immunoglobulins such as IgG, IgM, IgA, IgE and IgD. An example of a therapeutic analyte includes but is not limited to doxorubicin.

As used herein, the term "sample" refers to a biological sample or non-biological sample. Biological sample refers to a sample obtained from a biological subject, including a sample of biological tissue or fluid origin obtained *in vivo* or *in vitro.* Such samples can be, but are not limited to blood, blood plasma, serum, buccal smear, amniotic fluid, prenatal tissue, sweat, nasal swab or urine, organs, tissues, fractions, and cells isolated from mammals including humans. Biological samples also may include sections of the biological sample including tissues (e.g., sectional portions of an organ or tissue). Biological samples may also include extracts from a biological sample, for example, an antigen from a biological fluid (e.g., blood or urine). A non-biological sample includes but is not limited to water from some ecological niche, e.g., a river or a lake; or a solution used in a laboratory.

As used herein, the term "applicator" refers to a device to apply a compound onto a test strip. An applicator may be a membrane, including but not limited to porous membranes, such as a filter membrane. Examples of filter membranes include but are not limited to Fusion5 from Whatman™, glass fibers, symmetric and asymmetric polyethersulfone membranes, CytoSep™ and cellulose filter membranes.

As used herein, the term "porous membrane" refers to a membrane with pores. Examples of porous membrane include but are not limited to porous membranes may be selected from the group consisting of cellulose acetate, cellulosic paper, filter paper, tissue paper, writing paper, paper towel, cloth, or porous polymer film nitrocellulose membrane, polyvinylidene fluoride (PVDF) membrane and filter paper. In one embodiment, the porous membrane is a nitrocellulose membrane such as nitrocellulose acetate.

As used herein the terms "applied" or "contacted" may be used interchangeably with respect to the applicator.

As used herein, the term "nucleic acid" refers to any single or double-stranded RNA or DNA molecule, such as mRNA, cDNA, and genomic DNA.

As used herein, the term "oligonucleotide" refers to a single-stranded nucleotide polymer made of more than 2 nucleotide subunits covalently joined together. Preferably between 10 and 100 nucleotide units are present, most preferably between 12 and 50 nucleotides units are joined together. The sugar groups of the nucleotide subunits may be ribose, deoxyribose or modified derivatives thereof such as 2'-O-methyl ribose. The nucleotide subunits of an oligonucleotide may be joined by phosphodiester linkages, phosphorothioate linkages, methyl phosphonate linkages or by other rare or non-naturally-occurring linkages that do not prevent hybridization of the oligonucleotide. Furthermore, an oligonucleotide may have uncommon nucleotides or nonnucleotide moieties. An oligonucleotide as defined herein is a nucleic acid, preferably DNA, but may be RNA or have a combination of ribo- and deoxyribonucleotides covalently linked. Oligonucleotide probes and amplification oligonucleotides of a defined sequence may be produced by techniques known to those of ordinary skill in the art, such as by chemical or biochemical synthesis, and by in vitro or in vivo expression from recombinant nucleic acid molecules, e.g., bacterial or retroviral vectors. As intended by this disclosure, an oligonucleotide does not consist of wild-type chromosomal DNA or the in vivo transcription products thereof. One use of a probe is as a hybridization assay probe; probes may also be used as in vivo or in vitro therapeutic amplification oligomers or antisense agents to block or inhibit gene transcription, or translation in diseased, infected, or pathogenic cells.

As used herein, the term "peptide" refers to two or more amino acid molecules linked by amide bonds. Peptides may be naturally occurring or synthetic.

### BRIEF DESCRIPTION OF THE DRAWINGS

The invention will be better understood with reference to the detailed description when considered in conjunction with the non-limiting examples and the accompanying drawings, in which:

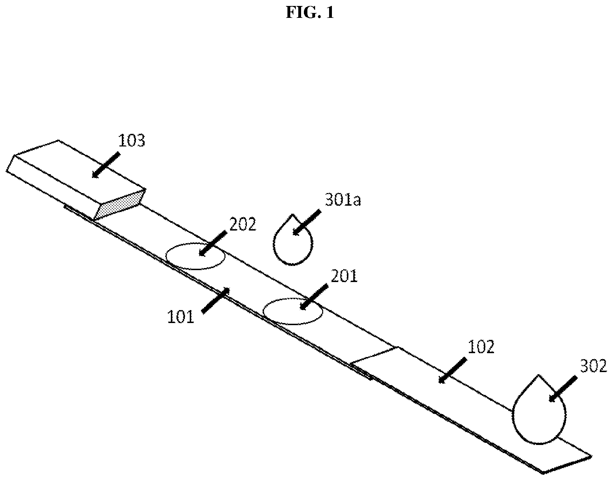

**Fig. 1****.** Schematic diagram illustrating the method to achieve paper-based ELISA colourimetric results via a combined wash and substrate addition step, i.e. no separate wash step is required. The first step entails the mixing of sample with the detector agent-enzyme conjugate and spotting onto the test spot/line **201.** This is followed by the second step of enzyme substrate **302** addition at substrate pad **102.** No waiting time is necessary between steps 1 and 2.

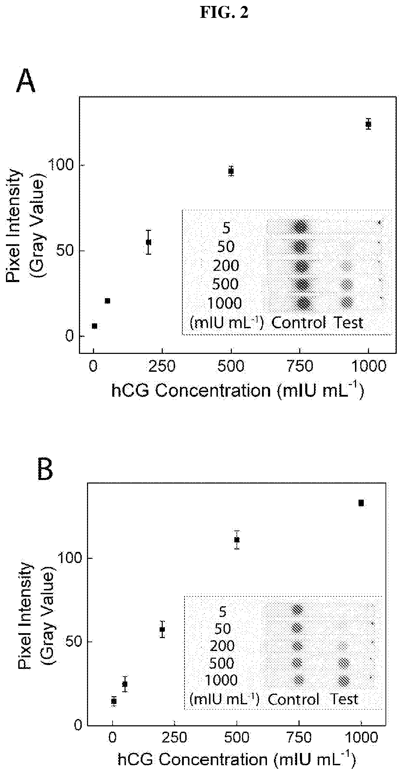

**Fig. 2****.** Graph of pixel intensity (gray value) against human chorionic gonadotropin (hCG) concentration for paper-based ELISA using (A) alkaline phosphatase with BCIP/NBT and (B) horseradish peroxidase with TMB. N=3. Representative images of the test strips are included as the inset images.

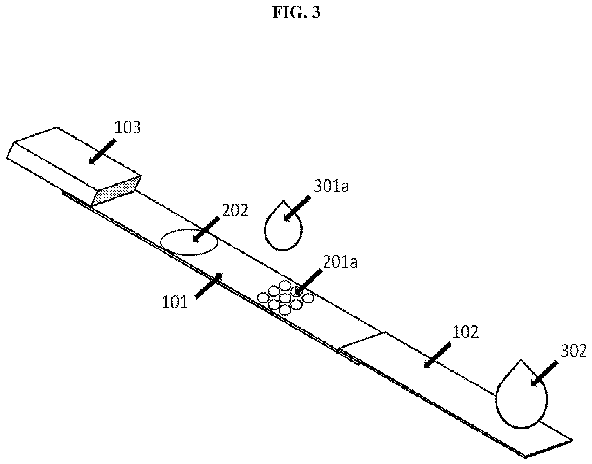

**Fig. 3****.** Schematic diagram illustrating the method to achieve an array of paper-based ELISA colourimetric results via a combined wash and substrate addition step, i.e. no separate wash step is required. The first step entails the mixing of sample with the detector agent-enzyme conjugate and spotting onto the test array **201a.** This is followed by the second step of enzyme substrate **302** addition at substrate pad **102.** No waiting time is necessary between steps 1 and 2.

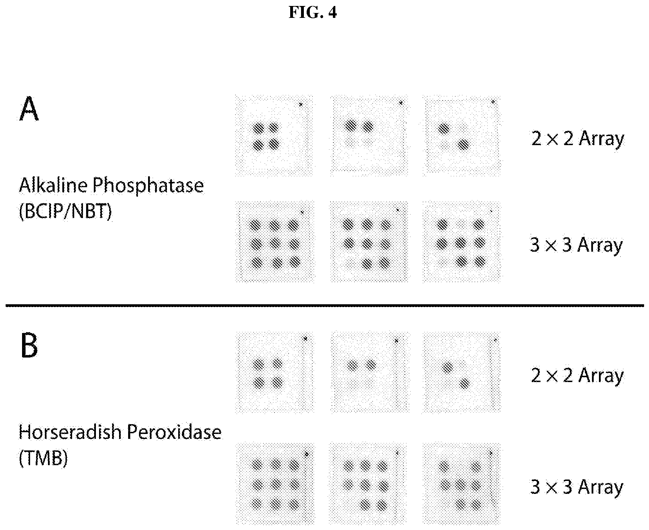

**Fig. 4****.** Representative images of arrays of paper-based ELISA colourimetric results obtained via a combined wash and substrate addition step, i.e. no separate wash step is required. 2 × 2 and 3 × 3 arrays obtained using (A) alkaline phosphatase with BCIP/NBT and (B) horseradish peroxidase with TMB. The darker and lighter results were obtained using 1000 mIU mL⁻1 and 50 mIU mL⁻1 of hCG respectively.

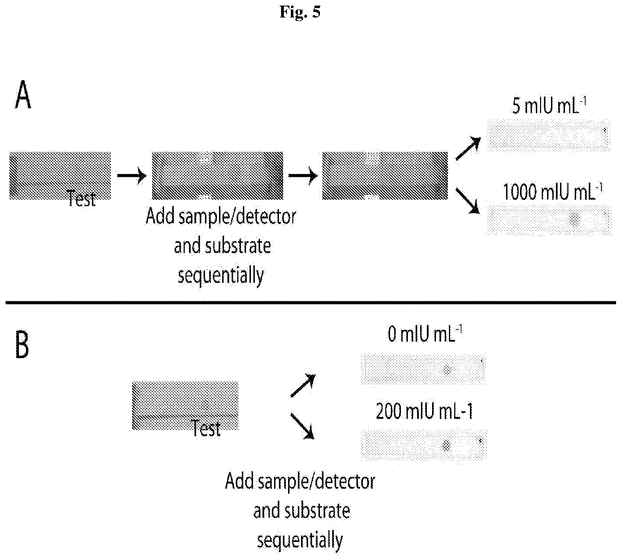

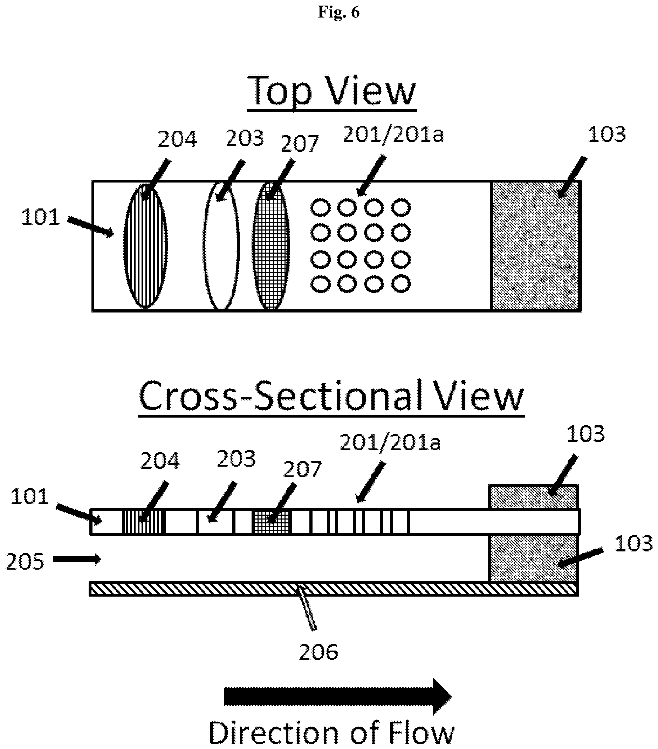

**Fig. 5****.** (**A**) Visualization of test spot using water-soluble dyes (blue food coloring in this example). The images are representative images of paper-based ELISA colorimetric results obtained using the combined wash and substrate addition step (i.e. no separate wash step was required). The AP and BCIP/NBT system was used along with the hCG assay, and with 15 min of substrate flow time. N = 3. (**B**) Visualization of test spot with water-insoluble dyes. The test spots were created using 3-µm yellow carboxylated polystyrene beads conjugated with capture antibodies. The images are representative images of paper-based ELISA colorimetric results obtained using the combined wash and substrate addition step (i.e. no separate wash step was required). The AP and BCIP/NBT system was used along with the luteinizing hormone assay, and with 15 min of substrate flow time. N = 3. NB: In Figure 6A, the substrate flow washed away the soluble dye, while in Figure 6B, the yellow color remained as the polystyrene beads remained entrapped within the test spot.
**Fig. 6****.** Schematic of alternative method for performing paper-based ELISA on a test strip.

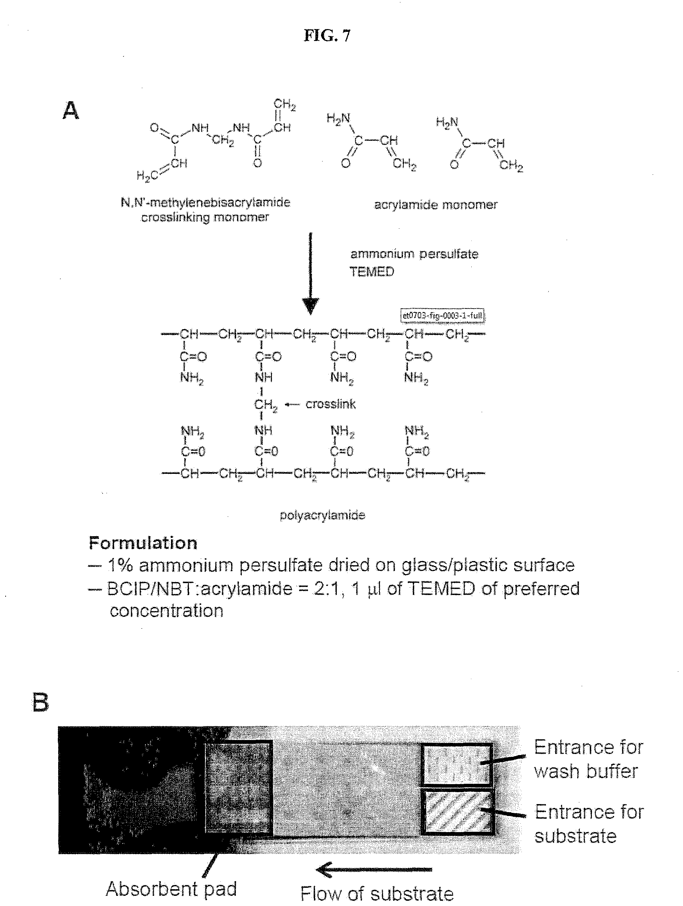

**Fig. 7****.** (A) Formulation of polyacrylamide-substrate mix for paper-based ELISA. (B) Image demonstrating application of polyacrylamide-substrate mix for paper-based ELISA.

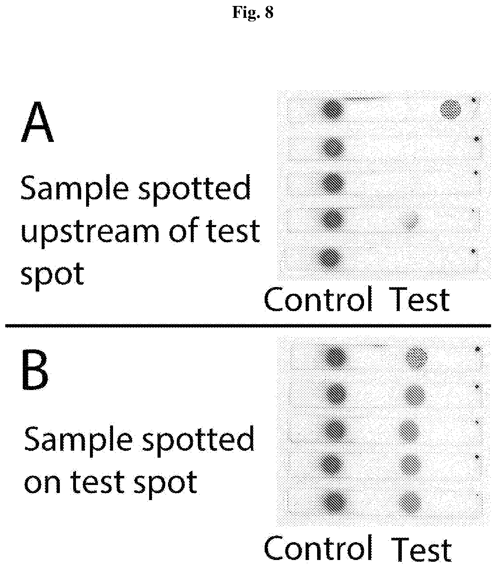

**Fig. 8****.** Images of the effect that sample spotting location has on the paper-based ELISA colourimetric results obtained via a combined wash and substrate addition step. The AP and BCIP/NBT system was used along with the hCG assay for a substrate flow time of 15 min. Sample concentration = 1000 mIU ml⁻¹. (A) Sample-detector mixture **301a** was posted upstream of the test spot (indicated by the granular dot). (B) Sample-detector mixture **301a** was spotted on the test spot (indicated by the granular dot). Nitrocellulose strips were treated using 2% BSA with 0.02% Tween®20. Anti-mouse AP conjugate was used as the control spot.

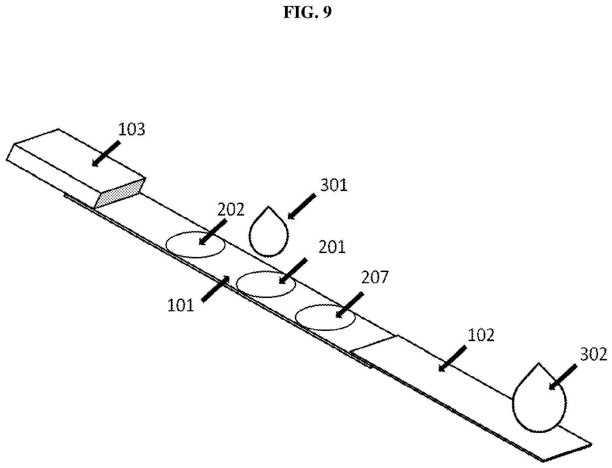

**Fig. 9****.** Schematic diagram illustrating prior art to achieving paper-based ELISA colourimetric results via a combined wash and substrate addition step (i.e. no separate wash step was required). The detector agent-enzyme conjugate was dried on the porous membrane **207** in this case. The first step entailed the introduction of the sample **301** only onto the test spot **201**, and the second step involved enzyme substrate **302** addition at substrate pad **102.** No waiting time was necessary between steps 1 and 2.

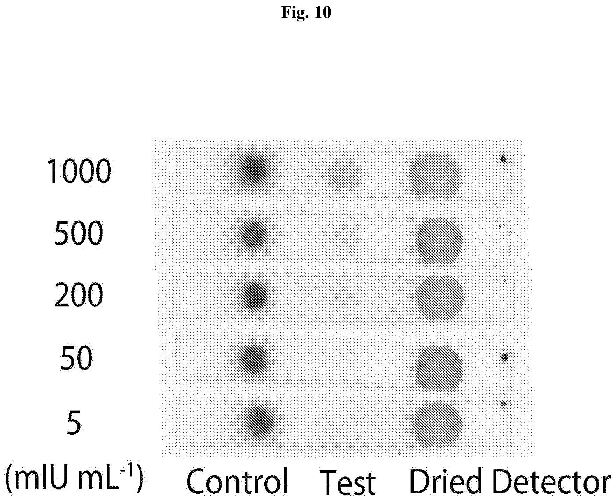

**Fig. 10****.** Representative images of paper-based ELISA colourimetric results obtained via prior art method of combined wash and substrate addition step (i.e. the detector agent-enzyme conjugate was dried on the porous membrane in this case after treating the nitrocellulose using 2% BSA with 0.02% Tween®20). The AP and BCIP/NBT system was used along with the hCG assay for a substrate flow time of 15 min. N=3. Anti-mouse AP conjugate was used as the control spot.

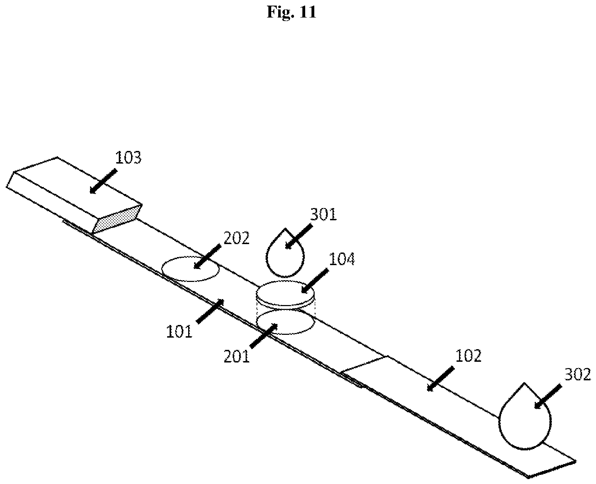

**Fig. 11****.** Illustration depicting the assembly of the conjugate pad **104** directly on top of the test spot/line **201.** NB: the conjugate pad **104** is intentionally separated from test spot/line **201** to reveal test spot/line **201.**

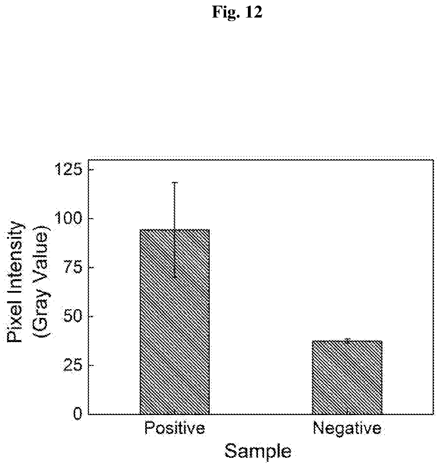

**Fig. 12****.** Pixel intensity (gray value) of colorimetric results obtained for HBsAg-positive and -negative "whole blood samples". The results were obtained from paper-based ELISA using RBC filter membranes as conjugate pads and assembled on top of the test spots. N = 3.

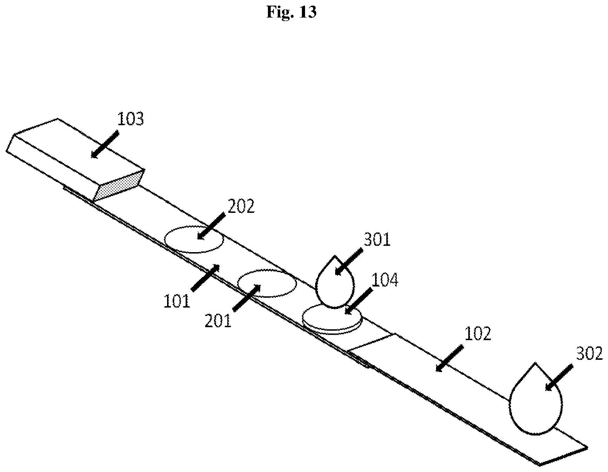

**Fig. 13****.** Illustration depicting the use of the conjugate pad **104** in front of the test spot/line **201.**

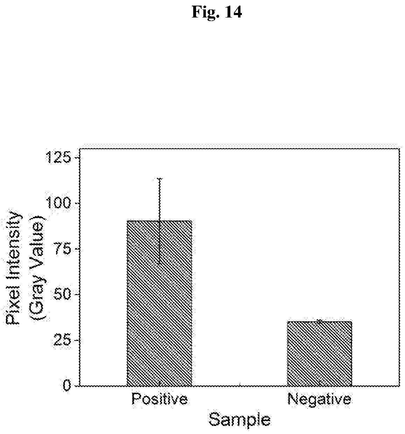

**Fig. 14****.** Pixel intensity (gray value) of colorimetric results obtained for HBsAg-positive and -negative "whole blood samples". The results were obtained from paper-based ELISA using RBC filter membranes as conjugate pads and assembled upstream of the test spots. N = 3.

### DETAILED DESCRIPTION OF THE PRESENT INVENTION

The invention is defined in the appended claims.

In one aspect, there is provided a test strip for a paper-based assay comprising, a substrate addition zone for receiving a substrate and a test zone, said test zone comprising a capture agent, wherein the substrate addition zone is located upstream of said test zone.

It will be generally understood that the substrate addition zone is located upstream of the test zone with respect to the direction of flow of liquid through and/or along said test strip.

In some embodiments, the substrate addition zone and the test zone are in a spaced part configuration. Spaced apart would be generally understood to mean that the substrate addition zone and test zone may be arranged anywhere on the test strip apart from each other, whilst maintaining the upstream location of the substrate addition zone with respect to the test zone. In other words, the test zone and substrate addition zone may be located on the test strip in any spaced apart relationship with respect to one another. For example, the substrate addition zone may be situated at one end or end region of the test strip and the test zone may be situated at the opposite end or end region of the test strip. The substrate addition zone and the test zone may also be situated in the middle region of the test strip.

In some embodiments, the substrate addition zone and the test zone are in an adjacent configuration.

In some embodiments, the substrate addition zone and the test zone are in an immediately adjacent configuration.

In some embodiments, the substrate addition zone and the test zone are situated on the test strip such that fluid flow may be direct from the substrate addition zone to the test zone. In other words, fluid flow from the substrate addition zone to the test zone is not interposed by any other zone or chemical addition, as is exemplified in Figure 1.

In other embodiments, the substrate addition zone and the test zone may be interposed by a detector zone.

In some embodiments, the sample may be premixed with a detector agent. It will be appreciated that the sample premixed with a detector agent may be contacted with the test strip at the test zone.

In some embodiments, the detector agent may be pre-dried onto the detector zone or test zone.

In some embodiments, the detector agent may be pre-dried onto an applicator. It will generally be understood that pre-drying refers to drying the detector agent onto the detector zone or test zone on the test strip or applicator prior to using the test strip. Drying the detector agent may take place in a vacuum or at atmospheric pressure. Drying the detector agent may also take place at ambient temperature or at temperatures above or below ambient temperature. It will also be appreciated that the detector agent may be dried at various levels of relative humidity. For example, 100%, 95%, 90%, 85%, 80%, 75%, 70%, 65%, 60%, 55%, 50%, 45%, 40%, 35%, 30%, 25%, 20%, 15%, 10%, 5%, 0%. It will generally be understood that the length of time of drying the detector agent will vary according to the conditions employed. The detector may also be dried with one or more additional compounds including but not limited to preservatives, proteins and/or surfactants. Examples of preservatives include but are not limited to sucrose and trehalose. An example of a protein that may be dried with the detector is bovine serum albumin (BSA). An example of a surfactant that may be dried with the detector is polysorbate 20. It will generally be appreciated that drying the detector with one or more additional compounds may confer stability during long-term storage.

In some embodiments, the detector agent may be pre-dried onto an applicator. It will be generally understood that an applicator may be any absorbent or porous material. For example, an applicator may be a membrane such as a filter membrane. It will also be generally understood that a membrane may be a single layer membrane or a plurality of layers. Examples of an applicator include but are not limited to Fusion5 from Whatman™, glass fibers, CytoSep™, symmetric and asymmetric polyethersulfone membranes, and cellulose filter membranes.

In one embodiment, an applicator may be a conjugate pad comprising a detector-agent conjugated to an enzyme.

It will be generally understood that the applicator may be untreated or treated prior to the addition of a detector agent. Pre-treatment of the applicator includes but is not limited to washing the applicator with compounds such as preservative, proteins and/or surfactants. Examples of preservative include but are not limited to sucrose and trehalose. Examples of proteins include but are not limited to bovine serum albumin (BSA). Examples of surfactants include but are not limited to polysorbate 20.

It will also be appreciated that the applicator may be cut to an appropriate size prior to use. The material, thickness, number of layers and size of the applicator used will be generally understood to vary based on the assay in question. The applicator will be applied onto the test zone or detector zone prior to the start of the assay.

In some embodiments, the sample may be contacted with the test strip at the test zone. In other embodiments, the sample is contacted with the test strip at the interposed detector zone.

In some embodiments, the sample may be applied onto the applicator.

In some embodiments, the applicator may be applied onto the test strip and the sample may be applied onto the applicator.

In some embodiments, the applicator may be removed from the test strip prior to addition of the substrate but after addition of the sample. The applicator may be removed after 1 s, 5 s, 10 s, 20 s, 30 s, 45 s, 1 min, 2 min, 3 min, 4 min, 5 min, 6 min, 7 min, 8 min, 9 min 10 min.

In other embodiments, the applicator may not be removed prior to addition of the substrate.

The detector agent may be selected from the group consisting of antibodies, antigens, oligonucleotides and peptides. It will be appreciated that the detector agent may be conjugated to a detectable material such as a coloured agent, a fluorescent agent, an enzyme or a chemiluminescent agent. For example, the detector agent may be conjugated to an enzyme selected from the group consisting of alkaline phosphatase, beta-galactosidase, glucose oxidase, glucose dehydrogenase and horseradish peroxidase. It will be appreciated that the detector agent will bind to an analyte of interest. It will also be appreciated that the enzyme conjugated on the detector agent will react with the substrate to emit a signal.

In one embodiment, the test strip is a porous membrane. Suitable porous membranes may be selected from the group consisting of cellulose acetate, cellulosic paper, filter paper, tissue paper, writing paper, paper towel, cloth, or porous polymer film nitrocellulose membrane, polyvinylidene fluoride (PVDF) membrane and filter paper. In one embodiment, the porous membrane is a nitrocellulose membrane such as nitrocellulose acetate.

In one embodiment, the test zone is subdivided into two or more areas, each area comprising the same or different capture agent. In some embodiments, each area may comprise one or more than one capture agent. In some embodiments, the test zone may occupy a footprint from a single test spot to an array of test spots 2 x 1, 2 x 2, 3 x 1, up to but not limited to 4 x 4, covering an area of up to 4 cm x 4 cm. The at least two or more areas may comprise the same or different capture agent. In some embodiments, the test strip may comprise more than one test zone. In other embodiments, the test strip may comprise an array of test zones, each test zone may occupy a footprint from a single test spot to an array of test spots 2 x 1, 2 x 2, 3 x 1, up to but not limited to 4 x 4, covering an area of up to 4 cm x 4 cm. In some embodiments, the capture agent may be selected from the group consisting of antibodies, antigens, oligonucleotides and peptides. It will be appreciated that the capture agent specifically binds to an analyte of interest and retains the analyte of interest on the test zone. In one embodiment, the or each capture agent may be mixed with a coloured marker to assist in the localization of the or each capture agent on said test strip. Examples of colour markers include but are not limited to dyes and coloured beads. It will be appreciated that dyes such as water dye, food dye, poster colour and colours that generically absorbs on porous membranes may be used. It will also be appreciated that coloured beads include but are not limited to dyed polystyrene beads, nanoparticles such as gold or silver nanoparticles and cerium nanoparticles.

In another embodiment, coloured beads may be mixed with the captured antibody and dispensed onto the test zone during the membrane preparation phase. An advantage of using coloured beads is that these produce a light, but observable colour to enable users to identify the test zone. A further advantage is that since coloured beads are used, the colour will persist throughout the experiment.

In one embodiment, the test strip further comprises one or more control areas located within or without of the test zone. The control area may be downstream or upstream of the test zone relative to the flow of the liquid through and/or along said test strip. The control area may be one or more spots in an array of test spots within the test zone.

In one embodiment, the test zone is marked on the test strip. Marking the test zone may assist the user to accurately dispense the mix onto the test zone. The test zone is marked on the test strip with a dye or an outline. It will generally be understood that the test zone may be marked by an indication line or circle. The dye may be a washable dye. For example, water dye, food dye, poster colour and colours that generically absorbs on porous membranes.

In some embodiments, the substrate may be an enzymatic substrate. The enzymatic substrate may be converted into a product that emits a signal. Suitable examples of such substrate include 5-Bromo-4-chloro-3-indolyl phosphate (BCIP), nitroblue tetrazolium (NBT) or combined BCIP/NBT, tetramethylbenzidine (TMB), diaminobenzidine (DAB), glucose/electron acceptor, glucose/potassium ferricyanide/Fe³⁺ and 5-bromo-4-chloro-3-indolyl-β-D-galactopyranoside (X-gal). It will be understood to a skilled person that other enzymatic substrates known in the art would be possible.

In one embodiment, the substrate may be contacted with the test strip as an aqueous solution. In another embodiment, the substrate may be contacted with the test strip as a polymer solution. It will generally be understood that a polymer solution refers to a polymeric solution prior to the polymeric solution setting into a gel. In yet another embodiment, the substrate may be contacted with the test strip in a polymer gel. It will generally be understood that the substrate may be added upstream at the substrate addition zone. It will also be appreciated that the substrate may be added via a cavity under the test strip. It will also be appreciated that the substrate may be added onto the test strip.

In some embodiments, the substrate is polymerized in polyacrylamide. In some embodiments, the substrate may be mixed with precast polymeric gels before flowing. The choice of polymeric gel includes, but not limited to, polyacrylamide gels. The advantage of applying gels to substrate is to improve the contact between the substrate and test zone. In addition, the curing of gels may consume the excess, unreacted substrate to improve the signal stability.

In some embodiments, the substrate may be dispensed onto the test strip. In other embodiments, the substrate may be dispensed onto the underside of the test strip. For example, the aqueous solution, polymeric solution or polymeric gel comprising the substrate may be dispensed onto the test strip. In another example, the aqueous solution, polymeric solution or polymeric gel comprising the substrate may be dispensed onto the underside of the test strip. In some embodiments, when the substrate is dispensed onto the underside of the test strip, this dispensing step is via a cavity underneath the test strip.

In some embodiments a sample is contacted with the test strip directly at the test zone. In one embodiment, a sample is dispensed onto said test zone. The sample may be one of blood, urine, saliva, sweat, nasal swab with or without a suitable buffer, and with or without a detector, or a mix of at least two of blood, urine, saliva, sweat, nasal swab with or without a suitable buffer, and with or without a detector. The sample may be added in the range of 0.1 to 100 µl. For example, 0.1 µl to 100 µl, 0.5 µl to 95 µl, 1 µl to 90 µl, 1.5 µl to 85 µl, 2 µl to 80 µl, 2.5 µl to 75 µl, 3 µl to 70 µl, 3.5 µl to 65 µl, 4 µl to 60 µl, 4.5 µl to 55 µl, 5 µl to 50 µl, 5.5 µl to 45 µl, 6 µl to 40 µl, 6.5 µl to 35 µl,7 µl to 30 µl, 7.5 µl to 30 µl, 8 µl to 25 µl, 8.5 µl to 20 µl, 9 µl to 15 µl, 9.5 µl to 10 µl.

In one aspect, there is provided a paper-based enzyme assay comprising a test strip as described herein.

In one aspect, there is provided a method of detecting the presence of an analyte in a sample, said method comprising the steps of: a) contacting said sample onto said test zone of a test strip as described herein, wherein said sample comprises a detector antibody specific for said analyte; b) dispensing a substrate onto the substrate addition zone; and c) detecting an emitted signal from said test zone to confirm the presence or absence of said analyte in said sample.

In one aspect, there is provided a method of detecting the presence of an analyte in a sample, said method comprising the steps of: a) contacting said sample onto said detector zone of a test strip as described herein,; b) dispensing a substrate onto the substrate addition zone; and c) detecting an emitted signal from said test zone to confirm the presence or absence of said analyte in said sample.

In some embodiments, the sample may be contacted onto the test zone or the detector zone directly.

In some embodiments, the sample may be contacted onto the test zone or detector zone via the applicator located at the test zone or detector zone.

In some embodiments, the applicator may be applied onto or contacted with the test zone. In other embodiments, the applicator may be applied onto or contacted with the detector zone. It will be appreciated that the sample may be added onto the applicator either in situ on the test strip or prior to contacting the test strip with the applicator.

It will be appreciated that the applicator may be removed prior to substrate addition. It will also be appreciated that the applicator may be removed at any time prior to the substrate front reaching a zone where the applicator was applied. For example, the test zone or the detector zone. For example, the applicator may be applied onto the test zone or detector zone and the sample may then be added onto the applicator. The applicator may then be removed prior to substrate addition, or after substrate is added but prior to the substrate front reaching the applicator. In another example, the applicator may be applied onto the test zone or detector zone. The applicator may then be removed and the sample may be added onto the zone from which the applicator was removed. Subsequently, substrate may be added.

The emitted signal may be detected by means of a colour change, a chemiluminescent detector such as a charge-coupled device (CCD) imager or X-ray or a plate reader. It will be appreciated that other methods of detection known in the art may be used. These include but are not limited to fluorescence, radioactive, electrochemiluminescence and electrochemical detection methods.

In one embodiment, step (b) may immediately follow step (a) with no waiting time in between steps (a) and (b). In another embodiment, step (a) may be followed by an incubation period prior to step (b). Incubation period may be from between 1 second to 10 minutes. For example, between 10 seconds to 10 minutes, between 20 seconds to 9 minutes, between 30 seconds to 8 minutes, between 30 seconds to 7 minutes, between 40 seconds to 6 minutes, between 50 seconds to 5 minutes, between 1 minute to 4 minutes, between 2 minutes to 3 minutes. In some embodiments, incubation of the test strip after step (a) permits the sample to interact with the capture agent at the test zone and increase the signal. In other embodiments, incubation of the test strip after step (a) permits the sample to interact with the detector agent at the detector zone and increase the signal. It will be generally understood that the incubation period may be varied depending on the sample and capture agent used.

In one embodiment, the signal is a colourimetric signal. In another embodiment, the signal is a fluorescent signal. In yet another embodiment, the signal is a chemiluminescent signal. It another embodiment, the signal is an electrochemiluminescent signal. In another embodiment, the signal is an electrochemical signal.

In some embodiments, the test strip may be washed after step (a). In other embodiments, the test strip may be washed after step (b). In yet other embodiments, the test strip may be washed after step (a) and after step (b).

The wash step can be introduced by a droplet of wash buffer onto the test membrane thus ensuring that excess unbound sample or substrate is washed downstream before the test zone contacts the substrate. The mentioned droplet may range from 10 µl to 500 µl. For example, 15 µl to 450 µl , 20 µl to 400µl, 25 µl to 350 µl, 30 µl to 350 µl, 35 µl to 300 µl, 40 µl to 250 µl, 45 µl to 200 µl, 50 µl to 150 µl, 55 µl to 100 µl, 60 µl to 100 µl, 70 µl to 90 µl or 80 µl to 90 µl.

Advantageously, the invention described herein provides improved paper-based assay results, including cleaner signal readout, reduction in false positives and/or false negatives and improved efficiency in obtaining results.

The invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

### EXPERIMENTAL SECTION

Non-limiting examples of the invention, including the best mode, and a comparative example will be further described in greater detail by reference to specific Examples, which should not be construed as in any way limiting the scope of the invention.

### EXAMPLE 1

### Method of performing paper-based ELISA

### Materials and Methods

### Paper-based Wash-free ELISA Procedure and Colourimetric Results using Nitrocellulose Membranes.

Prior to performing the paper-based wash-free ELISA, the nitrocellulose membrane strip **101** was prepared with a test spot/line **201** and control spot/line **202 (****Figure 1**). A test spot/line **201** was created by spotting anti-beta human chorionic gonadotropin (hCG) antibody (capture antibody) upstream of the strip and demarcating this spot using a pencil. A control spot/line **202** to confirm the validity of the assay, if desired, was created by spotting anti-mouse antibody-enzyme (AP or HRP, AP = alkaline phosphatase, HRP = horseradish peroxidase) further downstream of the test spot/line **201** unless other indicated. The strips were vacuum dried, blocked using 0.1× PBS blocking buffer with 1% BSA, 0.05% Tween®20, and then washed with 5 mM of HNa₂O₄P.2H₂O in double distilled (d.d.) H20, pH ∼ 7.6, unless otherwise indicated. Finally, the strips were vacuum dried again to allow complete removal of water. Next, the substrate pad **102** and absorbent pad **103** were overlapped sufficiently with the nitrocellulose membrane **101** to ensure continuous flow.

To start the ELISA (**Figure 1**), anti-alpha hCG antibody-AP or HRP conjugate was prepared at a desired concentration (preferably ∼ 2 µg mL⁻1). hCG ELISA standards (as samples) of different concentrations, 5, 50, 200, 500 and 1000 mIU mL⁻1, were added to this anti-alpha hCG antibody-AP or HRP conjugate at a ratio of 1:1 to form the sample-detector mixture. The sample-detector mixture **301a** was spotted onto the test spot/line **201** to form a detection complex with the capture antibody. Next, excess 5-bromo-4-chloro-3'-indolyphosphate/nitro-blue tetrazolium chloride (BCIP/NBT) or tetramethylbenzidine (TMB) substrate **302** was immediately added, and allowed to migrate across the nitrocellulose strip. A wash step is not necessary. Signal was recorded 12 min after addition of the substrate solution. The strips were then removed from the testing device, and washed with 1x Tris-buffered saline (TBS), 0.1% Tween 20. Finally, the strips were scanned in using a scanner to obtain digital images for image analysis.

### Results and Discussion

Distinct colorimetric results can be obtained, on the test spots, for the AP with BCIP/NBT system **(****Figure 2A**), and HRP with TMB system **(****Figure 2B**). For both enzymatic systems, a proportional relationship between intensity of the coloured result and hCG concentration can be observed at the lower concentrations, and the results appear to plateau at the higher concentrations.

### EXAMPLE 2

### Method of performing an array of paper-based ELISA

### Materials and Methods

### Paper-based Wash-free ELISA Array Procedure and Colourimetric Results using Nitrocellulose Membranes.

Prior to performing the array of paper-based wash-free ELISA, the nitrocellulose membrane strip **101** was prepared with an array of test spots **201a** and control spot/line **202** (**Figure 3**). An array of test spots **201a** was created by spotting anti-beta human chorionic gonadotropin (hCG) antibody (capture antibody) upstream of the strip and demarcating the spots using a pencil. A control spot/line **202** to confirm the validity of the assay, if desired, was created by spotting anti-mouse antibody-enzyme (AP or HRP) further downstream of the test spots **201a** unless otherwise indicated. The strips were vacuum dried, blocked using 0.1× PBS blocking buffer with 1% BSA, 0.05% Tween®20 , and then washed with 5 mM of HNa₂O₄P.2H₂O in double distilled (d.d.) H20, pH ∼ 7.6, unless otherwise indicated. Finally, the strips were vacuum dried again to allow complete removal of water. Next, the substrate pad **102** and absorbent pad **103** were overlapped sufficiently with the nitrocellulose membrane **101** to ensure continuous flow.

To start the ELISA (**Figure 3**), anti-alpha hCG antibody-AP or HRP (AP = alkaline phosphatase, HRP = horseradish peroxidase) conjugate was prepared at a desired concentration (preferably ∼ 2 µg mL⁻1). hCG ELISA standards (as samples) of different concentrations, 50 or 1000 mIU mL⁻1, were added to this anti-alpha hCG antibody-AP or HRP conjugate at a ratio of 1:1 to form the sample-detector mixture **301a.** The sample-detector mixture **301a** were spotted onto the test spots **201a** individually to form a detection complex with the capture antibody. Next, excess 5-bromo-4-chloro-3'-indolyphosphate salt/nitro-blue tetrazolium chloride (BCIP/NBT) or tetramethylbenzidine (TMB) substrate **302** was immediately added, and allowed to migrate across the nitrocellulose strip. A wash step is not necessary. Signal was recorded 12 min after addition of the substrate solution. The strips were then removed from the testing device, and washed with 1x Tris-buffered saline (TBS), 0.1% Tween 20. Finally, the strips were scanned in using a scanner to obtain digital images for image analysis.

### Results and Discussion

Distinct colourimetric results can be obtained, on the test spots, for the AP with BCIP/NBT system (**Figure 4A**), and HRP with TMB system (**Figure 4B**). For both enzymatic systems, colourimetric results were obtained for different 2 × 2 and 3 × 3 arrays. The 1000 mIU mL⁻1 samples gave a darker spot while the 50 mIU mL⁻1 samples gave a lighter spot. Results for the control spots were not shown in this example.

### EXAMPLE 3

### Visualizing of Test Spots using Dyes

Visualization of the test spots can be achieved using either water-soluble dyes or water-insoluble dyes (**Figure 5**). Water soluble dyes (e.g. food dyes) can be spotted onto the test spots (with or after the spotting of capture antibodies) to indicate where the sample-detector mixture should be introduced and/or where results should be expected (**Figure 5A**). Upon introduction of the substrate, the substrate flow washes away the water-soluble dye and colorimetric results can be observed on the test spots (depending on the sample concentration).

Water-insoluble dyes can also be spotted onto the test spots to indicate where the sample-detector mixture should be introduced and where results should be expected (**Figure 5B**). In this example, 3-µm carboxylated yellow polystyrene (PS) beads conjugated with capture antibodies were used as the water-insoluble "dyes" (NB: it might not be necessary for the capture antibodies to be conjugated with the beads). These beads permanently "stained" the test spots as the beads became entrapped within the porous membrane (bead size > pore size). Unlike the previous example, the beads did not get washed away by the substrate flow, and a different color change (yellow to brown in this example) could be observed on the test spots (depending on the sample concentration), even though the same enzymatic-substrate amplification system was used.

### EXAMPLE 4

### Alternative Method of performing paper-based ELISA

**Figure 6** demonstrates an alternative method to perform the paper-based ELISA. A sample-detector mixture **301a** is first introduced onto the test spot/line **201** or test spots **201a.** Next, the colourimetric substrate is introduced upstream before it flows underneath the porous test membrane and within cavity **205** after wash-buffer is introduced through the test strip **101** at region **204.** This introduces a separation between the substrate and the mix, thus ensuring that the unbound enzyme conjugate is washed downstream before the test zone **201/201a** contacts the substrate. The mentioned wash buffer droplet may range from 2 µl to 500 µl. Due to the hydrophilic nature of the device base **206,** the substrate distributes uniformly throughout the test spot within 2 sec. This ensures the enzyme-substrate reaction starts at about the same time.

In another embodiment, the enzyme conjugate is dried at region **207,** or anywhere between the test zone **201/201a** and region **203.** This allows for direct dispensing of biological samples **301** onto the test zone **201/201a** with minimum sample preparation. The colourimetric substrate then is introduced upstream before it flows underneath the porous test membrane and within cavity **205** after wash-buffer is introduced through the test strip **101** at region **204.**

Yet in another embodiment, the sample **301** or sample-detector mixture **301a** may be dispensed at Region **203** instead of the test zone **201/201a.** This technique is useful when the test requires a large amount of mix flows through the test zone. The amount of mix may range from 5 µl to 400 µl. The colourimetric substrate then is introduced upstream before it flows underneath the porous test membrane and within cavity **205** after wash-buffer is introduced through the test strip **101** at region **204.**

### EXAMPLE 5

### Achieving paper-based ELISA colourimetric results with precast polymeric gel-substrate mix.

As depicted in **Figure 6****,** the rationale for flowing substrate underneath the membrane is to ensure uniform distribution of the substrate in the shortest amount of time. Herein an approach to polymerize the substrate within 1 min is proposed. This ensures consistent contact of the substrate to the test zone.

To prepare the platform, 1% ammonium persulfate (dissolved in water) was coated and dried on the base **206** of the device (**Figure 6**). A piece of absorbent pad **103** and a piece of glass fiber membrane were adhered onto the nitrocellulose membrane downstream and upstream respectively using epoxy glue. A 3 x 3 array of 1 µl alkaline phosphatase (1 in 500 dilution) was dispensed on the nitrocellulose membrane. The setup was then dried in a vacuum oven for 1 hour.

To prepare the substrate mix, acrylamide and bis-acrylamide solution were first mixed at a ratio of 19:1, followed by dissolution in water to obtain a 30% solution (**Figure 7A**). Next, 1 part of this solution is mixed with 2 parts of BCIP/NBT, followed by addition of 1 µl of N,N,N',N'-tetramethylethylenediamine (TEMED) to obtain the final substrate mix. To conduct the experiment, 400 µl of wash buffer was added to the glass fiber membrane. When the nitrocellulose membrane was wet, 800 µl of substrate mix was dispensed into cavity **205.** Within 5 min, a blue colourimetric signal was observed in the area that was coated with alkaline phosphatase (**Figure 7B**). In practice, the wash buffer removed the unbound analytes and enzyme conjugates, and would wet the nitrocellulose membrane. The wetted nitrocellulose membrane ensured that the substrate would permeate through the membrane slowly, preventing smearing of the colourimetric results. This was advantageous, especially if the substrate did not precipitate properly upon enzyme-substrate reaction.

### EXAMPLE 6

### Limitations of prior art.

### Significance of Sample Location Spotting on Colourimetric Results.

**Figure 8A** shows colourimetric results obtained from spotting the sample-detector mixture **301a** upstream of the test spot/line **201.** This spotting location was chosen in replication of procedures in the art. **Figure 8B** highlights colourimetric results obtained from spotting the sample-detector mixture **301a** directly onto the test spot/line **201.** The results in **Figure 8A** highlighted the inconsistency and unreliability of the former approach whereby the sample-detector mixture were spotted upstream of the test spots. False negative results were obtained occasionally and the colouration within positive results was not evenly distributed. On the other hand, the colourimetric results obtained in **Figure 8B** appeared relatively more consistent and reliable. No false negative results were observed, and colouration within the positive results was more evenly distributed. This improves the performance in quantitative measurements. Spotting directly onto the test spot ensured sufficient time was available for binding between the capture antibody and the sample, and that the sample-detector mixture was evenly distributed before the sample bound to the capture antibody.

### Use of Impregnated Detector Enzyme Conjugate System

Prior to performing the paper-based ELISA, the nitrocellulose membrane was prepared with a test spot/line **201** and a control spot/line **202**, and an upstream region **207** was impregnated with dried detector antibody-AP conjugate (**Figure 9**). A test spot/line **201** was created by spotting anti-beta hCG antibody along the middle portion of the strip, and this spot was demarcated using a pencil. A control spot/line **202** to confirm the validity of the assay was created by spotting anti-mouse antibody-AP conjugate further downstream of the test spots. The strips were vacuum dried, blocked with a blocking buffer, and washed with 5 mM of HNa₂O₄P.2H₂O in d.d. H₂O, pH - 7.6. Finally, the strips were vacuum dried again to allow for complete removal of water. Anti-alpha hCG antibody-AP (∼20 µg mL⁻¹) in an aqueous solution containing 1% BSA, 5% sucrose and 5% trehalose was applied upstream of the test spots (**Figure 9**) at region **207**. The strips were again vacuum dried to allow for complete removal of water. Next, the substrate pad **102** and absorbent pad **103** were overlapped sufficiently with the nitrocellulose membrane to ensure continuous flow.

To start the ELISA (**Figure 9**), hCG standards **301** (as samples) of different concentrations, 5, 50, 200, 500 and 1000 mIU mL⁻¹, were spotted directly (without mixing with the detector antibody) onto the test spot/line **201**, and excess BCIP/NBT substrate **302** was immediately added to the substrate pad **102** (Note: no prior waiting or separate wash-step was required). After 15 min of substrate flow, the strips were removed from the testing device and washed with lx TBS, 0.1% Tween® 20. Finally, the strips were scanned using a scanner to obtain digital images.

### Results and Discussion

Colourimetric results were obtained from using this set of hCG concentrations (**Figure 10**). A directly proportional relationship was observed between the intensity of the test spot and the hCG concentration. The test spots were not uniform and appeared to have a lower intensity than the results in **Figure 2****.** The non-uniformity and lower colourimetric intensity were likely due to the uneven flow properties of the detector antibody-AP within the membrane, and its lower binding kinetics with captured hCG, respectively. (In Example 1, the detector antibody-enzyme and hCG were allowed to interact in the solution phase, and this has higher binding kinetics.) The intense colouration observed at region **207** where the detector antibody-AP conjugate was dried was due to non-specific adsorption of these conjugates to the membrane.

### EXAMPLE 7

### Conjugate Pad on Top of Test Spot/line

### Preparation of conjugate pad

RBC filter membranes (e.g. Fusion5 from Whatman) are first cut into desired diameters to handle the appropriate volume of whole blood samples. Although it is not necessary for the assay to work, pre-treatment of the filter membranes (e.g. with surfactants such as polysorbate 20) can be performed to reduce protein adsorption. Next, the desired detector agent-enzyme conjugate is dispensed onto these RBC filter membrane discs and dried. The desired detector agent-enzyme conjugate can be dried in the presence of sucrose and/or trehalose to preserve its stability during long-term storage. The filter membrane containing dried detector agent-enzyme is herein defined as conjugate pad **104.**

### Performing of paper-based ELISA

A representative method for assembly of the conjugate pad **104** with the test strip is illustrated in **Figure 11****.** In this method, conjugate pad **104** is assembled directly on top of and in contact with test spot/line **201.** (NB: The assay will also work if conjugate pad **104** is partially overlapping on top of test spot/line **201**) The paper platform test strip consists of overlapping porous materials: reaction matrix **101** (e.g. nitrocellulose), substrate pad **102** (e.g. glass fiber) and absorbent pad **103** (e.g. cellulose) where substrate pad **102** is considered as upstream and absorbent pad **103** as downstream of reaction matrix **101.** Reaction matrix **101** serves to create a test spot/line **201** and a control spot/line **202.** Test spot/line **201** is created by dispensing the desired capture agent (e.g. antibodies, aptamers, peptides, recombinant proteins) capable of capturing the desired analyte present in the sample **301** (e.g. whole blood, serum), while control spot/line **202** is created by dispensing a capture reagent (e.g. antibodies, aptamers, peptides, recombinant proteins) capable of capturing the detector agent (e.g. antibodies, aptamers, peptides, recombinant proteins)-enzyme (e.g. alkaline phosphatase, horseradish peroxidase, β-galactosidase) conjugate. Substrate pad **102** serves to transport the enzymatic substrate **302** (e.g. 5-bromo-4-chloro-3-indoyl phosphate/nitrobluetetrazolium, tetramethylbenzidine, 5-bromo-4-chloro-3-indolyl-β-D-galactopyranoside) into reaction matrix **101.** Absorbent pad **103** serves to wick up reagents from reaction matrix **101.** Transport of liquid reagents (e.g. enzymatic substrate **302**) through the porous materials (reaction matrix **101,** substrate pad **102** and absorbent pad **103**) occurs laterally and via capillary action.

In a preferred embodiment, performing the paper-based ELISA is initiated via direct introduction of sample **301** onto conjugate pad **104** and where sample **301** will dissolve the dried detector agent-enzyme conjugate. Any analyte of interest present in sample **301** will react with this conjugate to form an analyte-detector antibody complex. As conjugate pad **104** is in direct contact with test spot/line **201**, the non-particulate components in solution (e.g. detector agent-enzyme conjugate and any analyte-detector agent complex) from sample **301** will be filtered through conjugate pad **104**, and gets transferred directly onto test spot/line **201.** Any analyte-detector antibody complex present will bind with capture agents within test spot/line **201**. Enzymatic substrate **302** is introduced directly onto substrate pad **102**, after removal of conjugate pad **104**, and will migrate along substrate pad **102** and reaction matrix **101** before reaching absorbent pad **103**. (*NB: Removal of contact between conjugate pad 104 and reaction matrix **101** is crucial to prevent non-specific colorimetric smearing of reaction matrix **101**.)* Due to differential migration of components in enzymatic substrate **302**, any detector agent-enzyme conjugate not binding to test spot/line **201** will get washed downstream and binds to capture agents in control spot/line **202** before reacting with the enzymatic substrate components present in enzymatic substrate **302**. If the analyte is present in sample **301**, both test spot/line **201** and control spot/line **202** will eventually show colorimetric results; whereas if the analyte is not present in sample **301**, only the control spot/line **202** will eventually show colorimetric results.

### Hepatitis B surface antigen (HBsAg) paper-based ELISA using RBCs filter membranes as conjugate pads and assembled on top of the test spots

Briefly, the paper platform (nitrocellulose membrane) strip was prepared with a test spot by dispensing anti-hepatitis B surface antigen (HbsAg) antibody (capture antibody) upstream of the strip. The strips were vacuum dried, blocked using 1× PBS with 2% BSA and 0.02% Tween®20, and then washed with 5 mM of phosphate buffer in double distilled H₂O, pH ∼ 7.6. Finally, the strips were vacuum dried again to allow complete removal of water. "Whole blood samples" were obtained by mixing packed red blood cells with serum in the volume ratio of 1:1. Hepatitis B negative "whole blood sample" were obtained by using the mixed sample as it was. Hepatitis B positive "whole blood sample" were obtained by spiking recombinant HBsAg into the "whole blood sample" at a concentration of 4300 ng/mL. The detector component, anti-HBsAg antibody-AP conjugate, was dried on a red blood cell filter membrane (e.g. Fusion5) disc. The desired concentration was preferably ∼ 1 µg/mL, and dried in 1 mM Tris buffer pH ∼ 7.4 with 2% sucrose and 0.2% BSA. The red blood cell filter membrane containing the dried anti-HBsAg antibody-AP conjugate was termed the conjugate pad. This conjugate pad was directly assembled on top of the test spot before starting the HBsAg paper-based ELISA. To start the HBsAg paper-based ELISA, ∼ 5 µL of "whole blood sample" was first aspirated into the droppers before dispensing onto the conjugate pad. The conjugate pad was next removed (when sufficient serum samples have been transferred onto the paper platform strip) and excess BCIP/NBT substrate was immediately added upstream of the test spot. After 15 min of substrate addition, the strips were washed with 1× Tris-buffered saline, 0.1% Tween®20. Finally, the strips were scanned using a scanner to obtain digital images for image analysis using ImageJ.

Results for the HBsAg paper-based ELISA (using RBCs filter membranes as conjugate pads and assembled on top of the test spots) are shown in Figure 12 where the pixel intensity of colorimetric signals obtained from HBsAg-positive "whole blood sample" was significantly higher than the pixel intensity of colorimetric signals obtained from HBsAg-negative "whole blood sample".

### EXAMPLE 8

### Conjugate Pad Upstream of Test Spot/line

### Preparation of conjugate pad

RBC filter membranes (e.g. Fusion5 from Whatman) are first cut into desired diameters to handle the appropriate volume of whole blood samples. Although it is not necessary for the assay to work, pre-treatment of the filter membranes (e.g. with surfactants such as polysorbate 20) can be performed to reduce protein adsorption. Next, the desired detector agent-enzyme conjugate is dispensed onto these RBC filter membrane discs and dried. The desired detector agent-enzyme conjugate can be dried in the presence of sucrose and/or trehalose to preserve its stability during long-term storage. The filter membrane containing dried detector agent-enzyme is herein defined as conjugate pad **104.**

An alternative representative method for assembling the conjugate pad **104** with the test strip is illustrated in **Figure 13****.** In this method, conjugate pad **104** is assembled upstream of test spot/line **201** (no overlapping). The paper platform test strip consists of overlapping porous materials: reaction matrix **101** (e.g. nitrocellulose), substrate pad **102** (e.g. glass fiber) and absorbent pad **103** (cellulose), where substrate pad **102** is considered as upstream and absorbent pad **103** as downstream of reaction matrix **101.** Reaction matrix **101** serves to create a test spot/line **201** and a control spot/line **202.** Test spot/line **201** is created by dispensing the desired capture agent (e.g. antibodies, aptamers, peptides, recombinant proteins) capable of capturing the desired analyte present in sample **301** (e.g. whole blood, serum), while control spot/line **202** is created by dispensing a capture reagent (e.g. antibodies, aptamers, peptides, recombinant proteins) capable of capturing the detector agent (e.g. antibodies, aptamers, peptides, recombinant proteins)-enzyme (e.g. alkaline phosphatase, horseradish peroxidase, β-galactosidase) conjugate. Substrate pad **102** serves to transport the enzymatic substrate **302** (e.g. 5-bromo-4-chloro-3-indoyl phosphate/nitrobluetetrazolium, tetramethylbenzidine, 5-bromo-4-chloro-3-indolyl-β-D-galactopyranoside) into reaction matrix **101.** Absorbent pad **103** serves to wick up reagents from reaction matrix **101.** Transport of liquid reagents through the porous materials (reaction matrix **101,** substrate pad **102** and absorbent pad **103**) occurs laterally and via capillary action.

In a preferred embodiment, performing the paper-based ELISA is initiated via direct introduction of sample **301** onto conjugate pad **104**, and where sample **301** will dissolve the dried detector agent-enzyme conjugate. Any analyte of interest present in sample **301** will react with this conjugate to form an analyte-detector antibody conjugate. As conjugate pad **104** is in direct contact with reaction matrix **101**, the non-particulate components in solution (e.g. detector agent-enzyme conjugate and any analyte-detector agent complex) from sample **301** will filter through conjugate pad **104**, and gets transferred directly onto reaction matrix **101**. Enzymatic substrate **302** is introduced directly onto substrate pad **102** after removal of conjugate pad **104,** and will migrate along substrate pad **102** and reaction matrix **101** before reaching absorbent pad **103**. (*NB: Removal of contact between conjugate pad **104** and reaction matrix **101** is crucial to prevent non-specific colorimetric smearing of reaction matrix **101**.)* Due to differential migration of components in enzymatic substrate **302**, the detector agent enzyme complex or analyte-detector antibody conjugate will get washed downstream before reacting with the enzymatic substrate components present in enzymatic substrate **302**. During the downstream washing process, the analyte-detector antibody complex (if any) will bind with capture agents within test spot/line **201**, whereas the detector agent-enzyme conjugate will bind to capture agents in control spot/line **202**. If the analyte is present in sample **301**, both test spot/line **201** and control spot/line **202** will eventually show colorimetric results; whereas if the analyte is not present in sample **301**, only the control spot/line **202** will eventually show colorimetric results.

### Hepatitis B surface antigen (HBsAg) paper-based ELISA using RBCs filter membranes as conjugate pads and assembled upstream of the test spots

Briefly, the paper platform (nitrocellulose membrane) strip was prepared with a test spot by dispensing anti-hepatitis B surface antigen (HbsAg) antibody (capture antibody) upstream of the strip. The strips were dried, blocked using 1× PBS with 2% BSA with 0.02% Tween®20, and then washed with 5 mM of phosphate buffer in double distilled H₂O, pH ∼ 7.6. Finally, the strips were dried again to allow complete removal of water.

"Whole blood sample" was obtained by mixing packed red blood cells with serum in the volume ratio of 1:1. Hepatitis B negative "whole blood sample" was obtained by using the mixed sample as it is. Hepatitis B positive "whole blood sample" were obtained by spiking recombinant HBsAg into the "whole blood sample" at a concentration of 4300 ng/mL. The detector component, anti-HBsAg antibody-AP conjugate, was dried on a red blood cell filter membrane (e.g. Fusion5) disc. The desired concentration was preferably ∼ 1 µg/mL, and dried in 1 mM Tris buffer pH ∼ 7.4 with 2% sucrose and 0.2% BSA. The red blood cell filter membrane containing the dried anti-HBsAg antibody-AP conjugate was termed the conjugate pad. This conjugate pad was assembled upstream of the test spot (but downstream of the enzymatic substrate addition region) before starting the HBsAg paper-based ELISA. To start the HBsAg paper-based ELISA, ∼ 5 µL of "whole blood sample" was first aspirated into the droppers before dispensing onto the conjugate pad. The conjugate pad was next removed (when sufficient serum samples have been transferred onto the paper platform strip), and excess BCIP/NBT substrate was immediately added upstream of the test spot. After 15 min of substrate addition, the strips were washed with 1× Tris-buffered saline, 0.1% Tween® 20. Finally, the strips were scanned using a scanner to obtain digital images for image analysis using ImageJ.

Results for the HBsAg paper-based ELISA (using RBCs filter membranes as conjugate pads and assembled upstream of the test spots) are shown in **Figure 14** where the pixel intensity of colorimetric signals obtained from HBsAg-positive "whole blood sample" were significantly higher than the pixel intensity of colorimetric signals obtained from HBsAg-negative "whole blood sample".

## Claims

1. A test strip (101) for a paper-based assay comprising, a substrate addition zone (102) for receiving a substrate (302) and a test zone (201, 201a) for receiving a sample (301, 301a), **characterized in that** the sample is to be contacted at the test zone (201, 201a), said test zone (201, 201a) comprising a capture agent and wherein the substrate addition zone (102) is located upstream of said test zone (201, 201a).

2. The test strip (101) according to claim 1, wherein the substrate addition zone (102) and the test zone (201, 201a) are in a spaced apart configuration, optionally wherein the substrate addition zone (102) and the test zone (201, 201a) are in an adjacent configuration, optionally wherein the substrate addition zone (102) and the test zone (201, 201a) are in an immediately adjacent configuration, optionally wherein the substrate addition zone (102) and the test zone (201, 201a) are not interposed by any other zone, optionally wherein the substrate addition zone (102) and the test zone (201, 201a) are interposed by a detector zone (207).

3. The test strip (101) according to any one of claims 1 or 2, wherein the sample is premixed with a detector agent, optionally wherein the detector agent is selected from the group consisting of antibodies, antigens, oligonucleotides and peptides, and wherein the detector agent is conjugated to an enzyme, optionally wherein the enzyme is alkaline phosphatase, beta-galactosidase, glucose dehydrogenase and horseradish peroxidase.

4. The test strip (101) according to any one of claims 1 or 2, wherein a detector agent is pre-dried onto the test zone (201, 201a), or wherein a detector agent is pre-dried onto an applicator (104) and wherein the applicator (104) is to be applied onto the test zone (201, 201a).

5. The test strip (101) according to any one of claims 1 to 4, wherein the test strip (101) is a porous membrane, optionally wherein the porous membrane is selected from the group consisting of nitrocellulose membrane, polyvinylidene fluoride (PVDF) membrane and filter paper, optionally wherein the membrane is nitrocellulose membrane.

6. The test strip (101) according to any one of claims 1 to 5, wherein the test zone (201, 201a) is subdivided into two or more areas, each area comprising the same or different capture agent, optionally wherein the two or more areas comprises an array, optionally wherein the capture agent is selected from the group consisting of antibodies, antigens, oligonucleotides and peptides, optionally wherein the capture agent is mixed with a coloured marker to assist in the localization of the or each capture agent on said test strip (101).

7. The test strip (101) according to any one of claims 1 to 6, wherein the test strip (101) further comprises one or more control areas (202) located within or without of the test zone (201, 201a).

8. The test strip (101) according to any one of claims 1 to 7, wherein the test zone (201, 201a) is marked on the test strip (101), optionally wherein the test zone (201, 201a) is marked on the test strip (101) with a dye or outline, optionally wherein the dye is a washable dye.

9. The test strip (101) according to any one of claims 1 to 8, wherein the substrate is an enzymatic substrate, optionally wherein the enzymatic substrate is BCIP, NBT or combined BCIP/NBT, TMB, DAB, glucose/potassium ferricyanide/Fe3+ or X-gal.

10. The test strip (101) according to any one of claims 1 to 9, wherein the substrate is contacted with the test strip (101) in an aqueous solution, polymer solution or a polymer gel.

11. The test strip (201, 201a) according to any one of claims 1 to 10, wherein the substrate is dispensed on the underside of the test strip (101).

12. A paper-based enzyme assay comprising a test strip (101) as defined in any of claims 1 to 11.

13. A method of detecting the presence of an analyte in a sample, said method comprising the steps of:
d) contacting said sample onto said test zone (201, 201a) of a test strip (101) according to claim 1;
e) dispensing a substrate onto the substrate addition zone (102); and
f) detecting an emitted signal from said test zone (201, 201a) to confirm the presence or absence of said analyte in said sample.

14. The method of claim 13, wherein the signal is a colourimetric signal, optionally wherein the signal is a fluorescent signal, optionally wherein the signal is a chemiluminescent signal, optionally wherein the signal is an electrochemiluminescent signal, optionally wherein the signal is an electrochemical signal.

## Patentansprüche

1. Ein Teststreifen (101) für einen Papier-basierten Assay, umfassend eine Substratzugabezone (102) zur Aufnahme eines Substrats (302) und eine Testzone (201, 201a) zur Aufnahme einer Probe (301, 301a), **dadurch gekennzeichnet, dass** die Probe in der Testzone (201, 201a) in Kontakt gebracht wird, die Testzone (201, 201a) ein Einfangmittel umfasst und wobei die Substratzugabezone (102) stromauwärts der Testzone (201, 201a) lokalisiert ist.

2. Der Teststreifen (101) nach Anspruch 1, wobei die Substratzugabezone (102) und die Testzone (201, 201a) voneinander beabstandet sind, optional wobei die Substratzugabezone (102) und die Testzone (201, 201a) benachbart sind, optional wobei die Substratzugabezone (102) und die Testzone (201, 201a) direkt benachbart sind, optional wobei die Substratzugabezone (102) und die Testzone (201, 201a) nicht durch eine andere Zone unterbrochen werden, optional wobei die Substratzugabezone (102) und die Testzone (201, 201a) durch eine Detektorzone (207) unterbrochen werden.

3. Der Teststreifen (101) nach einem der Ansprüche 1 oder 2, wobei die Probe mit einem Detektormittel vorgemischt wird, optional wobei das Detektormittel ausgewählt ist aus der Gruppe bestehend aus Antikörpern, Antigenen, Oligonukleotiden und Peptiden und wobei das Detektormittel mit einem Enzym konjugiert ist, optional wobei das Enzym eine alkalische Phopsphatase, Beta-Galaktosidase, Glucosedehydrogenase und eine Meerrettich-Peroxidase ist.

4. Der Teststreifen (101) nach einem der Ansprüche 1 oder 2, wobei ein Detektormittel auf der Testzone (201, 201a) vorgetrocknet wird, oder wobei ein Detektormittel auf einem Applikator (104) vorgetrocknet wird und wobei der Applikator (104) auf die Testzone (201, 201a) aufgebracht wird.

5. Der Teststreifen (101) nach einem der Ansprüche 1 bis 4, wobei der Teststreifen (101) eine poröse Membran ist, optional wobei die poröse Membran ausgewählt ist aus der Gruppe bestehend aus Nitrocellulose Membranen, Polyvinylidenfluorid (PVDF) Membranen und Filterpapier, optional wobei die Membran eine Nitrocellulose Membran ist.

6. Der Teststreifen (101) nach einem der Ansprüche 1 bis 5, wobei die Testzone (201, 201a) in zwei oder mehrere Bereiche unterteilt ist, jeder Bereich die gleichen oder verschiedene Einfangmittel umfasst, optional wobei die zwei oder mehreren Bereiche einen Array umfassen, optional wobei das Einfangmittel ausgewählt ist aus der Gruppe bestehend aus Antikörpern, Antigenen, Oligonukleotiden und Peptiden, optional wobei das Einfangmittel mit einem farbigen Marker gemischt wird um die Lokalisation jedes Einfangmittels auf dem Teststreifen (101) zu unterstützen.

7. Der Teststreifen (101) nach einem der Ansprüche 1 bis 6, wobei der Teststreifen (101) weiterhin ein oder mehrere Kontrollbereiche (202) umfasst, die innerhalb oder außerhalb der Testzone (201, 201a) angeordnet sind.

8. Der Teststreifen (101) nach einem der Ansprüche 1 bis 7, wobei die Testzone (201, 201a) auf dem Teststreifen (101) markiert ist, optional wobei die Testzone (201, 201a) mit einem Farbstoff oder Umriss auf dem Teststreifen (101) markiert ist, optional wobei der Farbstoff ein waschbarer Farbstoff ist.

9. Der Teststreifen (101) nach einem der Ansprüche 1 bis 8, wobei das Substrat ein enzymatisches Substrat ist, optional wobei das enzymatische Substrat BCIP, NBT oder kombiniertes BCIP/NBT, TMB, DAB, Glukose/Kalium Ferricyanid/Fe³⁺ oder X-gal ist.

10. Der Teststreifen (101) nach einem der Ansprüche 1 bis 9, wobei das Substrat mit dem Teststreifen (101) in einer wässrigen Lösung, einer Polymerlösung oder einem Polymergel in Kontakt gebracht wird.

11. Der Teststreifen (201, 201a) nach einem der Ansprüche 1 bis 10, wobei das Substrat auf der Unterseite des Teststreifens (101) verteilt wird.

12. Ein Papier-basierter Enzymassay umfassend einen Teststreifen (101) wie in einem der Ansprüche 1 bis 11 definiert.

13. Ein Verfahren zur Detektion der Anwesenheit eines Analyts in einer Probe, umfassend die folgenden Schritte:
d) in Kontakt bringen der Probe auf der Testzone (201, 201a) eines Teststreifens (101) nach Anspruch 1;
e) verteilen des Substrats auf der Substratzugabezone (102); und
f) erfassen eines von der Testzone (201, 201a) ausgesendeten Signals um die Anwesenheit oder Abwesenheit des Analyts in der Probe zu bestätigen.

14. Das Verfahren nach Anspruch 12, wobei das Signal ein kolorimetrisches Signal ist, optional wobei das Signal ein fluoreszierendes Signal ist, optional wobei das Signal ein chemilumineszentes Signal ist, optional wobei das Signal ein elektrochemilumineszentes Signal ist, optional wobei das Signal ein elektrochemisches Signal ist.

## Revendications

1. Bandelette réactive (101) pour un dosage sur papier comprenant une zone d'ajout de substrat (102) pour recevoir un substrat (302) et une zone de test (201, 201a) pour recevoir un échantillon (301, 301a), **caractérisée en ce que** l'échantillon doit être mis en contact au niveau de la zone de test (201, 201a), ladite zone de test (201, 201a) comprenant un agent de capture et la zone d'ajout de substrat (102) étant située en amont de ladite zone de test (201, 201a).

2. Bandelette réactive (101) selon la revendication 1, dans laquelle la zone d'ajout de substrat (102) et la zone de test (201, 201a) sont dans une configuration espacée, éventuellement dans laquelle la zone d'ajout de substrat (102) et la zone de test (201, 201a) sont dans une configuration adjacente, éventuellement dans laquelle la zone d'ajout de substrat (102) et la zone de test (201, 201a) sont dans une configuration immédiatement adjacente, éventuellement dans laquelle la zone d'ajout de substrat (102) et la zone de test (201, 201a) ne sont pas séparées par une quelconque autre zone, éventuellement dans laquelle la zone d'ajout de substrat (102) et la zone de test (201, 201a) sont séparées par une zone de détection (207).

3. Bandelette réactive (101) selon l'une quelconque des revendications 1 ou 2, dans laquelle l'échantillon est prémélangé avec un agent de détection, éventuellement dans laquelle l'agent de détection est choisi dans le groupe constitué par les anticorps, les antigènes, les oligonucléotides et les peptides, et dans laquelle l'agent de détection est conjugué à une enzyme, éventuellement dans laquelle l'enzyme est la phosphatase alcaline, la bêta-galactosidase, la glucose déshydrogénase et la peroxydase de raifort.

4. Bandelette réactive (101) selon l'une quelconque des revendications 1 ou 2, dans laquelle un agent de détection est préséché sur la zone de test (201, 201a), ou dans laquelle un agent de détection est préséché sur un applicateur (104) et dans laquelle l'applicateur (104) doit être appliqué sur la zone de test (201, 201a).

5. Bandelette réactive (101) selon l'une quelconque des revendications 1 à 4, la bandelette réactive (101) étant une membrane poreuse, la membrane poreuse étant éventuellement choisie dans le groupe constitué par une membrane de nitrocellulose, une membrane de polyfluorure de vinylidène (PVDF) et un papier filtre, la membrane étant éventuellement une membrane de nitrocellulose.

6. Bandelette réactive (101) selon l'une quelconque des revendications 1 à 5, dans laquelle la zone de test (201, 201a) est subdivisée en au moins deux zones, chaque zone comprenant un agent de capture identique ou différent, éventuellement dans laquelle les au moins deux zones comprennent un réseau, éventuellement dans laquelle l'agent de capture est choisi dans le groupe constitué par les anticorps, les antigènes, les oligonucléotides et les peptides, éventuellement dans laquelle l'agent de capture est mélangé avec un marqueur coloré pour faciliter la localisation du ou de chaque agent de capture sur ladite bandelette réactive (101).

7. Bandelette réactive (101) selon l'une quelconque des revendications 1 à 6, la bandelette réactive (101) comprenant en outre une ou plusieurs zones témoins (202) situées à l'intérieur ou sans de la zone de test (201, 201a).

8. Bandelette réactive (101) selon l'une quelconque des revendications 1 à 7, la zone de test (201, 201a) étant marquée sur la bandelette réactive (101), la zone de test (201, 201a) étant éventuellement marquée sur la bandelette réactive (101) avec un colorant ou un contour, le colorant étant éventuellement un colorant lavable.

9. Bandelette réactive (101) selon l'une quelconque des revendications 1 à 8, dans laquelle le substrat est un substrat enzymatique, éventuellement dans laquelle le substrat enzymatique est le BCIP, le NBT ou le BCIP/NBT combiné, la TMB, la DAB, le glucose/ferricyanure de potassium/Fe3+ ou le X-gal.

10. Bandelette réactive (101) selon l'une quelconque des revendications 1 à 9, le substrat étant mis en contact avec la bandelette réactive (101) dans une solution aqueuse, une solution polymère ou un gel polymère.

11. Bandelette réactive (201, 201a) selon l'une quelconque des revendications 1 à 10, le substrat étant appliqué sur le dessous de la bandelette réactive (101).

12. Dosage enzymatique sur papier comprenant une bandelette réactive (101) telle que définie dans l'une quelconque des revendications 1 à 11.

13. Procédé de détection de la présence d'un analyte dans un échantillon, ledit procédé comprenant les étapes consistant à :
d) mettre ledit échantillon en contact sur ladite zone de test (201, 201a) d'une bandelette réactive (101) selon la revendication 1 ;
e) appliquer un substrat sur la zone d'ajout de substrat (102) ; et
f) détecter un signal émis depuis ladite zone de test (201, 201a) pour confirmer la présence ou l'absence dudit analyte dans ledit échantillon.

14. Procédé de la revendication 13, dans lequel le signal est un signal colorimétrique, éventuellement dans lequel le signal est un signal fluorescent, éventuellement dans lequel le signal est un signal chimioluminescent, éventuellement dans lequel le signal est un signal électrochimioluminescent, éventuellement dans lequel le signal est un signal électrochimique.
